(19) Europäisches Patentamt
European Patent Office
Office européen des brevets

(11) Publication number: 0 200 818 A1

(12) # EUROPEAN PATENT APPLICATION

(21) Application number: 85114937.7

(22) Date of filing: 26.11.85

(51) Int. Cl.⁴: C 07 D 407/14, A 61 K 31/35

(30) Priority: 06.12.84 JP 256573/84

(43) Date of publication of application: 12.11.86 Bulletin 86/46

(84) Designated Contracting States: AT BE CH DE FR GB IT LI LU NL SE

(71) Applicant: MICROBIAL CHEMISTRY RESEARCH FOUNDATION, 14-23, Kamiosaki 3 Chome Shinagawa-ku, Tokyo 141 (JP)

(72) Inventor: Umezawa, Hamao, Prof., 23, Toyotama-kita 4-chome, Nerima-ku Tokyo (JP)
Inventor: Takeuchi, Tomio, 1-11, Higashigotanda 5-chome, Shinagawa-ku Tokyo (JP)
Inventor: Sawa, Tsutomu, 6-7, Ryosei 4-chome, Ayase-city Kanagawa Prefecture (JP)
Inventor: Naganawa, Hiroshi, 17, Denenchofu-honcho 3-chome, Ohta-ku Tokyo (JP)
Inventor: Isshiki, Kunio, 2-13-1332, 3910, Oba, Fujisawa-city Kanagawa Prefecture (JP)
Inventor: Kanbayashi, Nobuo, 3-24, Namiki 1-chome, Tsuchiura-city Ibaraki Prefecture (JP)
Inventor: Morishima, Hajime, 1-32, Nakameguro 1-chome, Meguro-ku Tokyo (JP)

(74) Representative: Becker, Heinrich Karl Engelbert, Dr. et al, HOECHST AKTIENGESELLSCHAFT Central Patent Department P.O. Box 80 03 20, D-6230 Frankfurt am Main 80 (DE)

(54) **Derivatives of the pluramycin group having anti-tumor activity, process for their preparation and their use as medicaments.**

(57) The present invention relates to novel derivatives of antibiotics of the pluramycin group and a process for producing the same. The compounds exhibit strong anti-tumor activity and can, therefore, by used as medicaments.

EP 0 200 818 A1

The present invention relates to novel derivatives of antibiotics of the pluramycin group that exhibit anti-tumor activity as well as a method of producing the same.

Three antibiotics of the pluramycin group are known to be obtained from cultures of actinomycetes, and these antibiotics are:

TITLE MODIFIED
see front page

kidamycin of the formula (II):

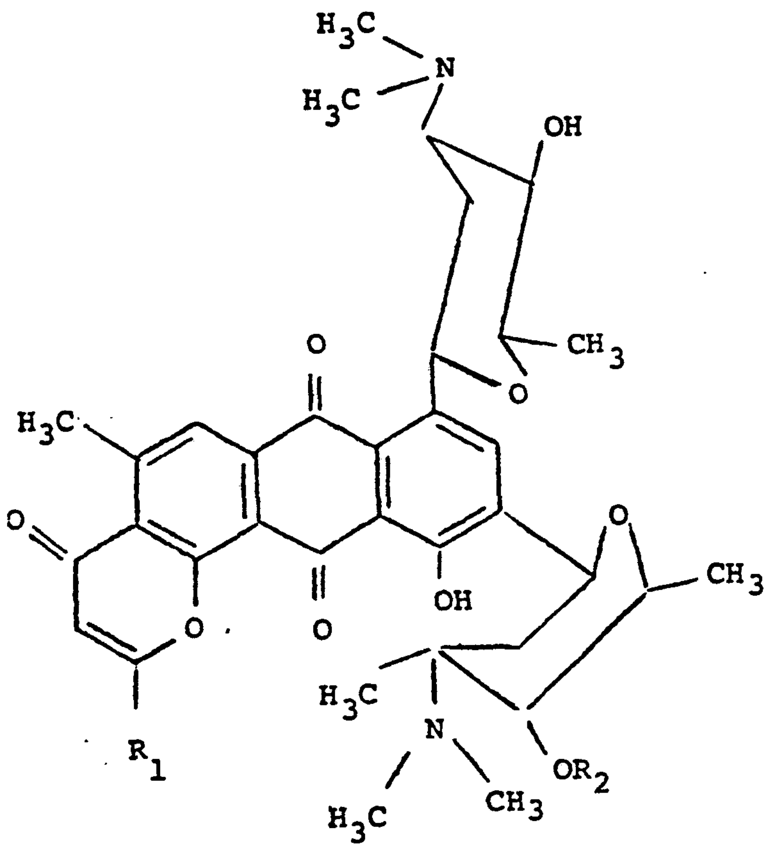

(II)

wherein $R_1$ and $R_2$ are each $-\underset{\substack{|\\ CH_3}}{C}=CH-CH_3$ or a hydrogen atom (Mr. Furukawa et al.: Tetrahedron, <u>31</u>, 2989 - 2995, 1975);

pluramycin of the same formula (II) wherein $R_1$ and $R_2$ are each $-\underset{\substack{|\\ CH_3}}{C}\overset{O}{\wedge}C-CH=CH-CH_3$ or an acetyl group (S. Kondo et al: J. Antibiotics, <u>30</u>, 1143 - 1145, 1977);

neopluramycin of the formula (II) wherein $R_1$ and

$R_2$ are each $-\underset{CH_3}{\underset{|}{C}}=CH-CH_3$ or an acetyl group (S. Kondo et al.: J. Antibiotics, <u>30</u>, 1143 - 1145, 1977).

These antibiotics exhibit strong action to inhibit the growth of cultured leukemia cell P 388 and intensive R&D efforts have been made to use these antibiotics as cancer control agents.

As mentioned above, the antibiotics of the pluramycin group exhibit strong anti-tumor activity <u>in vivo</u> and have been closely studied to exploit their potential use as cancer control agents. However, these antibiotics themselves cause kidney toxicity and other adverse side effects in laboratory animals and it has been desired to mitigate such adverse side effects.

Therefore, it has been searched for a variety of derivatives of the pluramycin group antibiotics that would have reduced side effects while retaining the high anti-tumor activity of such antibiotics. As a result, it has been found that compounds of the formula (I)

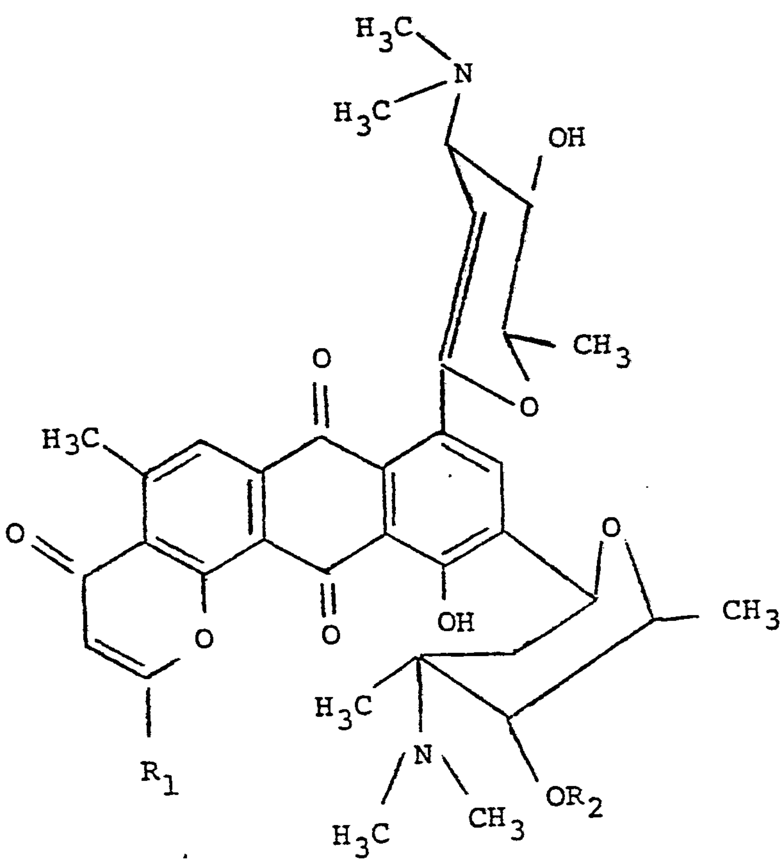

(I)

wherein $R_1$ is $-\underset{CH_3}{C}=CH-CH_3$ or $-\underset{CH_3}{\overset{O}{\overbrace{C-CH}}}-CH=CH-CH_3$;

$R^2$ is a hydrogen atom or an acetyl group, provided that when $R^2$ is a hydrogen atom, $R_1$ is $-\underset{CH_3}{C}=CH-CH_3$ show reduced side effects although they are somewhat inferior to the corresponding known antibiotics of the pluramycin group in terms of <u>in vivo</u> anti-tumor activity. The inventors have also found that these compounds of formula (I) can be readily obtained by illuminating solutions or suspensions of kidamycin, pluramycin or neopluramycin in inert solvents with ultraviolet radiation. The present invention has been accomplished on the basis of these findings.

The compounds in accordance with the present invention are novel and different in chemical structure from the starting pluramycin group antibiotics in that they have 5,6-dehydroangolosamine obtained by dehydrogenation at 5 and 6 positions in the angolosamine group of a specific pluramycin group antibiotic.

The compounds recited in claim 1 are hereunder designated as noted below depending upon the antibiotics from which they are originated.

| $R_1$ in formula (1) | $R_2$ in formula (I) | Designation |
|---|---|---|
| $-\underset{CH_3}{C}=CH-CH_3$ | -H | 5',6'-dehydro-kidamycin |
| $-\underset{CH_3}{C}=CH-CH_3$ | $-COCH_3$ | 5',6'-dehydro-neopluramycin |
| $-\underset{CH_3}{\overset{O}{\overbrace{C-CH}}}-CH=CH-CH_3$ | $-COCH_3$ | 5',6'-dehydro-pluramycin |

The compounds of formula (I) in accordance with the present invention have the following effects and advantages.

A. Anti-tumor activity

A suspension of 1 x $10^5$ L 1210 leukemia cells was transplated intraperitoneally in each of the $CDF_1$ mice tested. Thereafter, 5',6'-dehydrokidamycin was injected intraperitoneally for 10 days in predetermined amounts, 10, 5, 2.5, 1.25, 0.625 and 0.3125 mg/kg.

The treated mice were observed for 30 days following the injection and the percent life prolongation of each mouse was checked, with the number of days for which control mice administered physiological saline survived being taken as 100.

| Dose mg/kg/day | Percent life prolongation |
|---|---|
| 10 | 153 |
| 5 | 173 |
| 2.5 | 133 |
| 1.25 | 133 |
| 0.625 | 133 |
| 0.3125 | 147 |

B. Acute toxicity ($LD_{50}$)

The $LD_{50}$ of 5',6'-dehydrokidamycin for a single intraperitoneal injection in mice was 100 mg/kg.

C. Cytotoxicity

The growth of cultured leukemia cell L 1210 was inhibited by 0.07 μg/ml ($IC_{50}$) of 5',6'-dehydrokidamycin.

As shown above, the compound of the present invention exhibits strong anti-tumor activity against cultured leukemia cell L 1210 while causing low toxicity. Therefore,

the compound is expected to be useful as a cancer control agent.

The compounds recited in claim 1, namely, 5',6'-dehydrokidamycin,5',6'-dehydropluramycin, and 5',6'-dehydroneopluramycin can be produced in high yields from kidamycin, pluramycin and neopluramycin, respectively by irradiation with ultraviolet light.

For example, these starting antibiotics may be dissolved or suspended in inert solvents and exposed to ultraviolet radiation so as to convert them to the respective end compounds. The resulting compounds may be isolated in pure form from the reaction system by any of the known isolation and purification methods.

The ultraviolet treatment may be performed with a UV irradiator, Tomy Seiko TV 40 (Tomy Seiko Co., Ltd.) which is positioned 30 cm away from the target and irradiates UV light (100 volts and 15 watts) for 1 - 2 hours at room temperature.

The intensity and duration of UV irradiation are properly determined by the type of inert solvent used and the concentration of starting antibiotic but are by no means critical for the purposes of the present invention. With the intensity of UV radiation, temperature and duration of irradiation given in the previous paragraph, the concentration of a particular starting antibiotic may be set in the range of 250 - 1,000 μg/ml.

The present invention is hereunder described in greater detail by reference to Examples.

Example 1

Production of 5',6'-dehydrokidamycin :

A solution of kidamycin (5 mg) in methanol (20 ml) was placed in a petri dish (ID: 8.5 cm) and illuminated with UV radiation for 2 hours from Tomy Seiko TV 40 (100 volts, 15 watts) 30 cm away from the petri dish. Methanol was distilled off under vacuum and subjected to high-performance liquid chromatography on Nucleosil $_5C_{18}$ column

(20 mm$^{\varnothing}$ x 300 mm, Macherey & Nagel) using an acetonitrile-0.1M phosphate buffer (3:7) at pH 3.0 as a mobile phase. The eluate was adjusted to pH 7.0 with sodium bicarbonate and subjected to extraction with chloroform. After destilling off the chloroform under vacuum, the residue was dissolved in methanol and loaded onto a Sephadex LH-20 column (15 mm$^{\varnothing}$ x 170 mm). The column was eluted with methanol and the active fractions were collected and evaporated to dryness under vacuum to produce 3.6 mg of 5',6'-dehydrokidamycin. mp. 165 - 167°C (decomposed).

IR ($\nu_{max}^{KBr}$) $cm^{-1}$: 3420, 2965, 2930, 2860, 2820, 2775, 1645, 1635, 1605, 1580, 1465, 1435, 1420, 1375, 1310, 1270, 1230, 1090, 1050, 840

NMR ($CDCl_3$) δ :
13.9 (s, 1H), 7.93 (s, 1H), 7.72 (s, 1H), 7.47 (q, 1H), 6.38 (s, 1H), 5.43 (t, 1H), 5.00 (d, 1H), 4.33 (dq, 1H), 4.08 (dq, 1H), 3.66 (t, 1H), 3.45 (dd, 1H), 3.42 (d, 1H), 3.02 (s, 3H), 2.40 (s, 3H), 2.30 (dd, 1H), 2.25 (s, 3H), 2.23 (dd, 1H), 2.03 (d, 3H), 2.02 (s, 3H), 1.45 (d, 3H), 1.42 (d, 3H), 0.83 (s, 3H)

<u>Example 2</u>

Production of 5',6'-dehydropluramycin:

Pluramycin (5.2 mg) was subjected to 1-hr UV irradiation as in Example 1 to produce 2 mg of 5',6'-dehydropluramycin.

NMR ($CDCl_3$) δ :
13.7 (s, 1H), 7.98 (s, 1H), 7.78 (s, 1H), 6.52 (s, 1H), 6.05 (dq, 1H), 5.52 (dd, 1H), 5.41 (m, 1H), 5.15 (d, 1H), 4.97 (d, 1H), 4.3 (m, 2H), 4.12 (d, 1H), 3.77 (t, 1H), 3.67 (dd, 1H), 3.10 (s, 3H), 2.58 (s, 3H), 2.40 (s, 3H), ~2.6 (1H), 2.2 (1H), 2.18 (s, 3H), 1.88 (dd, 3H), 1.82 (s, 3H), 1.55 (d, 3H), 1.43 (d, 3H), 0.96 (s,3H)

Example 3

Production of 5',6'-dehydroneopluramycin:

Neopluramycin (10.8 mg) was subjected to 1-hr UV irradiation as in Example 1 to produce 2.8 mg of 5',6'-dehydroneopluramycin.

NRM ($CDCl_3$) δ :

13.8 (s, 1H), 7.93 (s, 1H), 7.78 (s, 1H), 7,46 (q, 1H), 6.38 (s, 1H), 5.53 (dd, 1H), 5.15 (d, 1H), 4.97 (d, 1H), 4.30 (m, 2H), 3.73 (t, 1H), 3.57 (dd, 1H), 3.02 (s, 3H), ~ 2.6 (1H), 2.49 (s, 3H), 2.37 (s, 3H), 2.2 (1H), 2.18 (s, 3H), 2.03 (d, 3H), 2.01 (s, 3H), 1.54 (d, 3H), 1.42 (d, 3H), 0.96 (s, 3H).

Claims:

1. A compound of the formula (I):

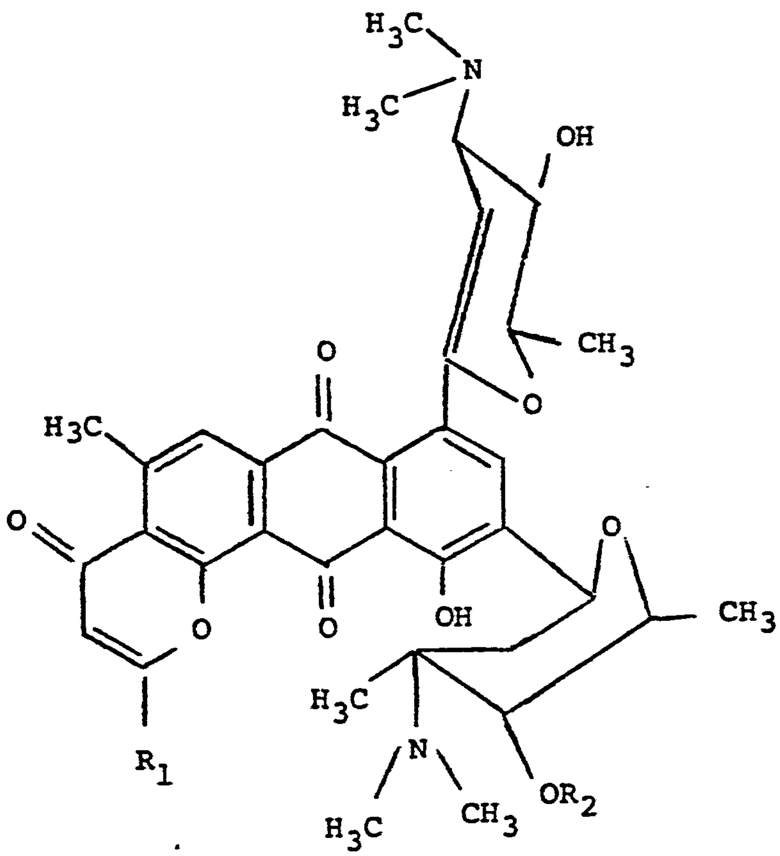

(I)

wherein $R_1$ is $-\underset{\large CH_3}{\underset{|}{C}}=CH-CH_3$ or $-\underset{\large CH_3}{\underset{|}{\overset{O}{C}}}-CH-CH=CH-CH_3$;

$R^2$ is a hydrogen atom or an acetyl group, provided that when $R^2$ is a hydrogen atom, $R_1$ is $-\underset{\large CH_3}{\underset{|}{C}}=CH-CH_3$.

2. A process for producing a compound of the formula (I)

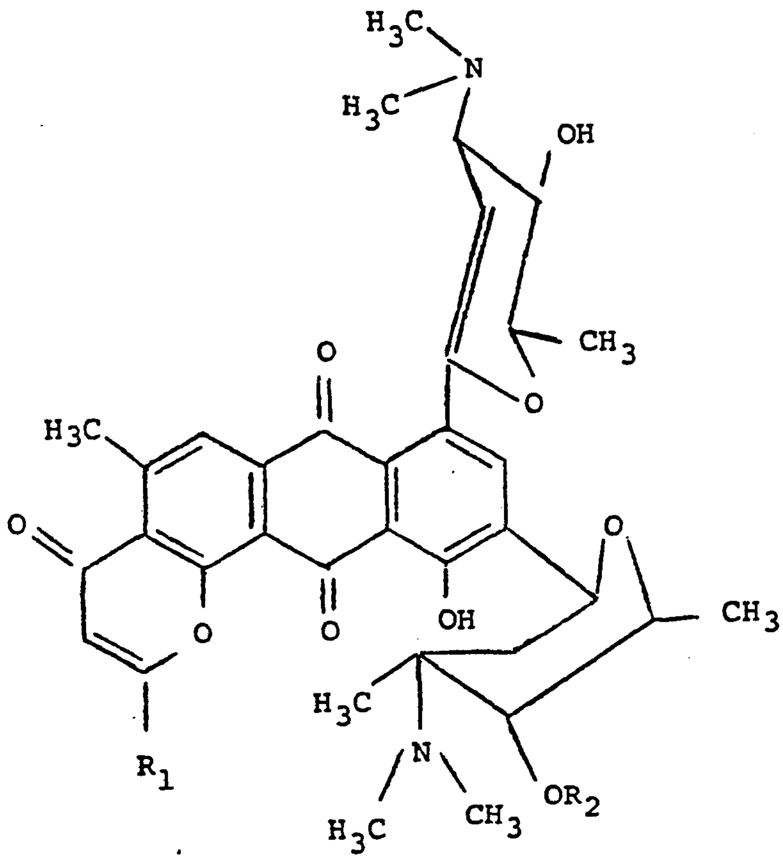

(I)

wherein $R_1$ is $-\underset{CH_3}{\underset{|}{C}}=CH-CH_3$ or 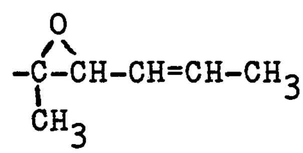;
$R^2$ is a hydrogen atom or an acetyl group, provided that when $R^2$ is a hydrogen atom, $R_1$ is $-\underset{CH_3}{\underset{|}{C}}=CH-CH_3$ which comprises irradiating an antibiotic of the formula (II):

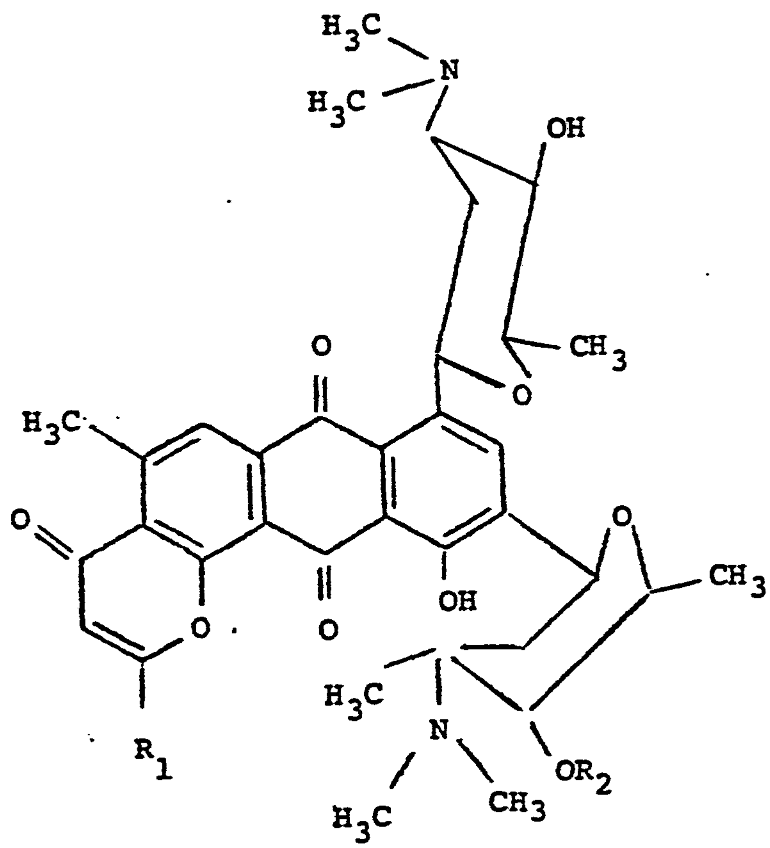

(II)

wherein $R_1$ and $R_2$ are the same as defined above with ultraviolet light.

3. A pharmaceutical composition which comprises as active ingredient a compound of the formula I as defined in claim 1 and a pharmaceutically acceptable carrier.

4. Use of a compound of the formula I as claimed in claim 1 for the preparation of a medicament having anti-tumor activity.

Claim for the contracting state Austria:

1. A process for producing a compound of the formula (I)

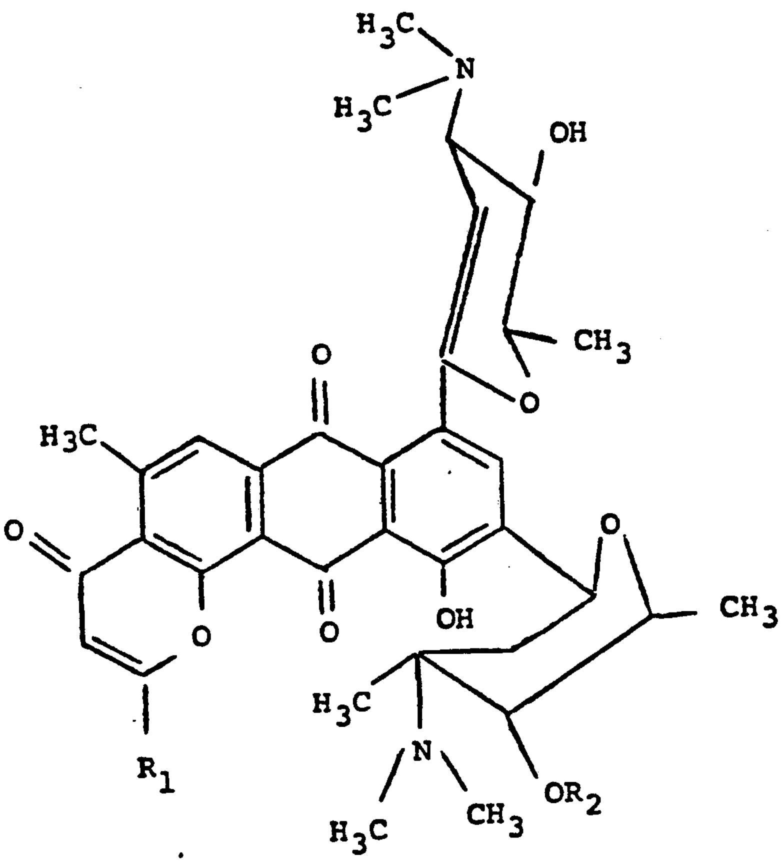

(I)

wherein $R_1$ is $-\underset{CH_3}{\underset{|}{C}}=CH-CH_3$ or $-\underset{CH_3}{\underset{|}{\overset{O}{\overset{\wedge}{C-CH}}}}-CH=CH-CH_3$;

$R^2$ is a hydrogen atom or an acetyl group, provided that when $R^2$ is a hydrogen atom, $R_1$ is $-\underset{CH_3}{\underset{|}{C}}=CH-CH_3$ which comprises irradiating an antibiotic of the formula (II):

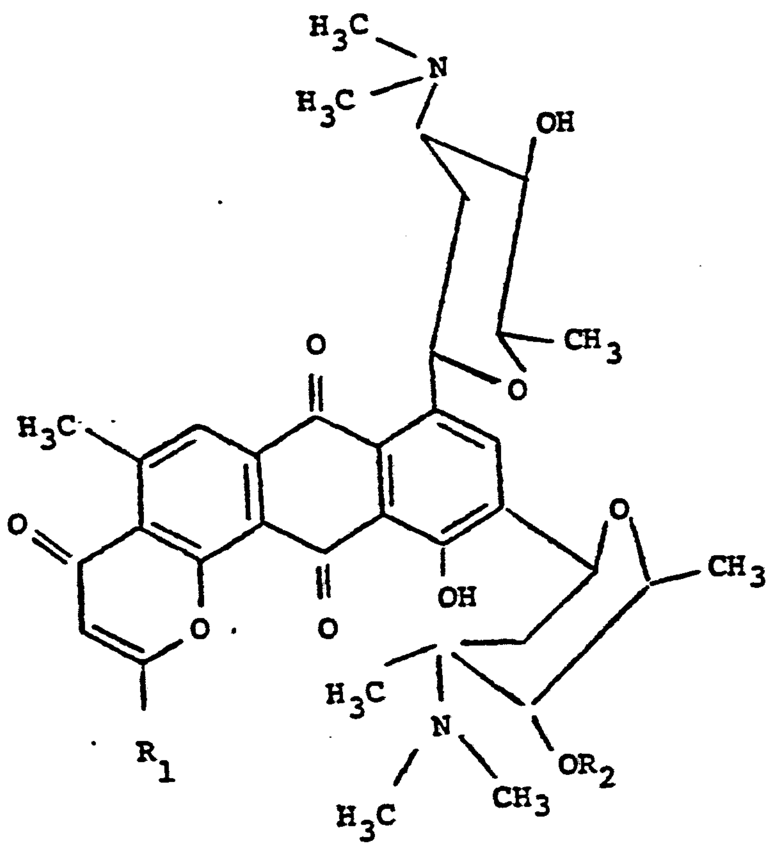

(II)

wherein $R_1$ and $R_2$ are the same as defined above with ultraviolet light.

0200818

European Patent Office

# EUROPEAN SEARCH REPORT

Application number

EP 85 11 4937

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
| --- | --- | --- | --- |
| X | THE JOURNAL OF ANTIBIOTICS, vol. 38, no. 2, February 1985, pages 236-241, Japan Antibiotics Research Association, Tokyo, JP; A. FREDENHAGEN et al.: "The photodeactivation of hedamycin, an antitumor antibiotic of the pluramycin type" * Pages 236-241 and reference 1) * | 1-3 | C 07 D 407/14<br>A 61 K 31/35 |
| X | HELVETICA CHIMICA ACTA, vol. 68, fasc. 2, 1985, pages 391-402, Schweizerische Chemische Gesellschaft, CH; A. FREDENHAGEN et al.: "The structures of some products from the photodegradation of the pluramycin antibiotics hedamycin and kidamycin" * Pages 391-402 and reference [1] * | 1,3 | TECHNICAL FIELDS SEARCHED (Int. Cl.4)<br>C 07 D 417/00<br>A 61 K 31/00 |
| Y,D | THE JOURNAL OF ANTIBIOTICS, vol. 30, no. 12, 1977, pages 1143-1145; S. KONDO: "Structures of pluramycin A and neopluramycin" * Page 1144 * | 1,3 | |

---  -/-

The present search report has been drawn up for all claims

| Place of search | Date of completion of the search | Examiner |
| --- | --- | --- |
| THE HAGUE | 17-06-1986 | VERHULST W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

0200818

Application number

EP 85 11 4937

Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y,D | TETRAHEDRON, vol. 31, 1975, pages 2989-2995, Pergamon Press, Oxford, GB; M. FURUKAWA et al.: "Structure and chemistry of kidamycin" * Page 2989 * | 1,3 | |
| | ----- | | |
| | | | TECHNICAL FIELDS SEARCHED (Int. Cl.4) |

The present search report has been drawn up for all claims

| Place of search | Date of completion of the search | Examiner |
|---|---|---|
| THE HAGUE | 17-06-1986 | VERHULST W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82